# EUROPEAN PATENT APPLICATION

(11) **EP 4 847 061 A1**
(43) Date of publication of application: **02.12.2026**
(21) Application number: 24919487.9
(22) Date of filing: 26.03.2024
(51) Int. Cl.: A61M 5/20

(54) **FIXED-DOSE AUTO-INJECTOR PEN**

(30) Priority: 25.01.2024 CN 202410103585
(71) Applicant: Eases Medical Technology (Suzhou) Co., Ltd, Suzhou, Jiangsu 215000 (CN)
(72) Inventor: LIU, Kai, Suzhou, Jiangsu 215000 (CN)
(74) Representative: Valet Patent Services Limited
(86) International application number: PCT/CN2024/083752
(87) International publication number: WO 2025/156396

(57) **Abstract**

Disclosed is a fixed-dose auto-injector pen, including: a pen barrel and a pen body arranged inside the pen barrel. One end of the pen body is provided with a pen cap, one end of the pen barrel is provided with a barrel cap, the other end of the pen body is provided with a rear cover, and the rear cover is provided with a protective bolt. A pre-filled syringe (PFS) sleeve container is arranged inside the pen body, and the PFS sleeve container is snap-fitted to the pen body. A PFS sleeve is slidably arranged in the PFS sleeve container, and a PFS is arranged in the PFS sleeve. The PFS is provided with a soft protective cap. A secondary power assembly is arranged between the PFS sleeve and the rear cover. The pen body is provided with a trigger protection mechanism, and the trigger protection mechanism is located between the pen cap and the PFS sleeve container. After an injection is completed, the trigger protection mechanism rebounds and locks, such that an exposed needle is shielded, thereby preventing the needle from remaining exposed to the outside, which may easily cause harm to users.

## Description

### TECHNICAL FIELD

The present invention relates to the technical field of injector pens, and more specifically, the present invention relates to a fixed-dose auto-injector pen.

### BACKGROUND ART

An allergic reaction is an emergency condition that may occur rapidly in everyday life (at the fastest, within a few minutes after contact with an allergen) and may even be life-threatening. It may be caused by a wide variety of allergens or triggering factors, which commonly include certain foods, insect stings, medicaments and latex. The symptoms produced by an allergic reaction vary, but symptoms involving the skin (urticaria, itching, reddening of the skin) are the most common. Most cases further involve swelling of the lips and tongue as well as of the respiratory tract (tightness of the throat, shortness of breath). An allergic reaction may also involve the gastrointestinal system (nausea, stomach ache, vomiting, diarrhoea), the cardiovascular system (accelerated heartbeat, chest pain, low blood pressure) or the central nervous system (headache, confusion).

The use of adrenaline for emergency treatment of anaphylaxis can constrict the blood vessels so as to increase blood pressure, relax the smooth muscle of the lungs so as to reduce wheezing and improve breathing, stimulate the heart (increase the heart rate) and reduce the urticaria and swelling that may occur on the face and around the lips. According to national food-allergy guidelines, adrenaline is the only recommended first-line therapeutic drug for allergic reactions. An anaphylactic episode is a sudden event, and where a potentially life-threatening allergic reaction is involved, an emergency plan needs to be drawn up in advance. Such a plan includes avoiding known allergens, recognizing the signs and symptoms of an allergic reaction, keeping an emergency auto-injector pen ready to be carried on one's person at all times, and immediately seeking emergency medical assistance.

At present, adrenaline injection solution in ampoules is relatively common in clinical practice and is very inexpensive, but it is rather cumbersome to use, which is disadvantageous for clinical emergency rescue. A pre-filled adrenaline product, by contrast, is an adrenaline pen in which a certain quantity of adrenaline is stored in an auto-injector; it is quick to use, and in an emergency situation a patient can even administer the injection to themselves. However, with existing pre-filled pens, the needle is still exposed to the outside after the injection has been completed, which readily causes injury to the person using the pen. It is therefore necessary to propose a fixed-dose auto-injector pen so as to at least partly solve the problems existing in the prior art.

### SUMMARY OF THE INVENTION

A series of concepts in simplified form are introduced in this Summary of the Invention, and these will be described in further detail in the Detailed Description of Embodiments. This Summary of the Invention is not intended to define the key features and essential technical features of the claimed technical solution, nor is it intended to determine the scope of protection of the claimed technical solution.

In order to at least partly solve the above problems, the present invention provides a fixed-dose auto-injector pen, including:
a pen barrel, a pen body being provided inside the pen barrel, one end of the pen body being provided with a pen cap, one end of the pen barrel being provided with a barrel cap, the other end of the pen body being provided with a rear cover, and the rear cover being provided with a protective bolt;
a pre-filled syringe (PFS) sleeve container being provided in the pen body, the PFS sleeve container being snap-fitted to the pen body, a PFS sleeve being slidably provided in the PFS sleeve container, a PFS being provided in the PFS sleeve, the PFS being provided with a soft protective cap, a secondary power assembly being provided between the PFS sleeve and the rear cover, and a trigger protection mechanism being provided on the pen body, the trigger protection mechanism being located between the pen cap and the PFS sleeve container.

Preferably, the barrel cap is provided with a gear, the pen body is provided with a rack, and the gear meshes with the rack;
the end of the pen barrel remote from the barrel cap is provided with a hook, the hook being snap-fitted to a retaining slot of the pen cap; a plurality of cap hooks are evenly provided inside the pen cap, and the cap hooks engage with the soft protective cap.

Preferably, an inner wall of the rear cover is provided with an insertion post, and two arc-shaped pieces are symmetrically provided on the inner wall of the rear cover, the two adjacent arc-shaped pieces being located on either side of the insertion post; the side of each arc-shaped piece close to the insertion post is provided with a protrusion, and the side of each arc-shaped piece remote from the insertion post is provided with two limiting pieces.

Preferably, the secondary power assembly includes:
a power assembly body, the power assembly body being slidably connected to an inner wall of the pen body, the end of the power assembly body remote from the rear cover being provided with a recess, the bottom end of an inner wall of the recess being provided with a sliding hole, the power assembly body being provided with two openings, a rear auxiliary spring being provided between the power assembly body and the rear cover, a clamp being provided in the power assembly body, and a piston push rod being provided in the clamp;
the clamp includes:
   a connecting portion, the connecting portion being slidably connected in the recess, the connecting portion being fixedly snap-fitted to the PFS sleeve, the connecting portion being provided with a sliding portion, the sliding portion extending into the sliding hole, an outer wall of the sliding portion being provided with two protrusion slots, protrusions being engaged in the protrusion slots, a push rod hole being provided in the sliding portion, an inner wall of the push rod hole being provided with two first slider slots, an inner side wall of each first slider slot being provided with a second slider slot, and an inner-wall inclined surface of the second slider slot being a guiding inclined surface;
   the piston push rod includes:
      a push rod, the push rod being slidably connected in the push rod hole, an outer wall of the push rod being provided with two sliding blocks, the sliding blocks being located in the second slider slots, a push rod groove being provided in the push rod, the insertion post extending into the push rod groove, a main spring being provided between the push rod groove and the rear cover, and the main spring being fitted over the insertion post; and
      a piston rod, one end of the piston rod being connected to the push rod, and the other end of the piston rod passing through the connecting portion and extending into the PFS.

Preferably, the PFS sleeve container includes:
a container body, the PFS sleeve being located in the container body, one end of the container body being provided with a cylindrical barrel, the other end of the container body being provided with a container hook, the container hook being snap-fitted to the pen body, the container body being provided with two first stop blocks, two arc-shaped elastic pieces being symmetrically provided on the container body, and the arc-shaped elastic pieces being provided with second stop blocks;
two steps being provided, above and below, where the tail end of the container body joins the tail end of the container hook, and the steps engaging with inner steps inside the pen body;
elastic-piece retaining slots, two elastic-piece retaining slots being symmetrically provided on the container body, the elastic-piece retaining slots being located between the cylindrical barrel and the container hook, an elastic-piece positioning groove being provided in each elastic-piece retaining slot, a support-platform groove being provided in each elastic-piece retaining slot, and an elastic piece being provided in each elastic-piece retaining slot;
two semicircular holes, each narrower at the top and wider at the bottom, being provided in each elastic-piece retaining slot;
the elastic piece includes:
   a support platform, the support platform being rotatably connected within the semicircular hole, which is narrower at the top and wider at the bottom, inside the elastic-piece retaining slot by means of a rotating shaft, one end of the support platform extending into the support-platform groove, and the other end of the support platform being provided with a support arm and a negative-angle catch platform.

Preferably, the trigger protection mechanism includes:
a protective shell slidably connected inside the pen body, the protective shell being located between the secondary power assembly and the pen cap, the protective shell including:
a shell body, one end of the shell body being provided with a through hole, the shell body being provided with a plurality of catch-platform holes, the catch-platform holes being adapted to the negative-angle catch platforms, the other end of the shell body being symmetrically provided with two arc-shaped trigger plates, each arc-shaped trigger plate being provided with a first rectangular hole, a second rectangular hole and a third rectangular hole arranged side by side, the arc-shaped trigger plates abutting against the power assembly, the first stop blocks being located in the third rectangular holes, and the second stop blocks being located in the first rectangular holes;
the container body being located between the two adjacent arc-shaped trigger plates;
a front auxiliary spring, the front auxiliary spring being provided inside the shell body, the front auxiliary spring being located between the inner wall of the shell body and the container body, and the front auxiliary spring being arranged close to the through hole.

Preferably, the protective bolt includes:
a bolt body, the bolt body being of a cap-shaped structure, an outer wall of the bolt body being provided with two catch tabs, the catch tabs being racetrack-shaped, the surfaces of the catch tabs being provided with anti-slip patterns, and the bolt body being connected to the pen body by means of the catch tabs; and
protective pieces, two protective pieces being symmetrically provided on an inner wall of the bolt body, the protective pieces being located between the power assembly body and the arc-shaped pieces, and the protective pieces being further located between two adjacent limiting pieces.

Preferably, the protective bolt is provided with a warning sticker, and the pen body is provided with a plurality of pen body labels.

Preferably, a drug viewing window is provided on the pen body, the container body is provided with an observation hole, and the position of the observation hole corresponds to that of the drug viewing window.

Preferably, the end of the pen barrel remote from the barrel cap and the pen cap are each provided with a detection hole, and the positions of the detection holes in the pen barrel and in the pen cap correspond to each other.

Compared with the prior art, the present invention at least has the following advantageous effects:
1. With the fixed-dose auto-injector pen according to the present invention, the pen body is withdrawn by opening the barrel cap; during withdrawal, the pen cap is detached by the pen barrel. The protective bolt is then pulled out and the trigger protection mechanism is pressed, whereupon the trigger protection mechanism triggers the secondary power assembly. The movement of the secondary power assembly drives the PFS sleeve to slide inside the PFS sleeve container, so that the PFS is pushed out and the injection is carried out. After the injection has been completed, the trigger protection mechanism rebounds and locks, thereby shielding the protruding needle and preventing the needle from remaining exposed to the outside, which would readily cause injury to the user.
2. At the same time as the pen barrel is opened, the pen body of the injector pen is automatically pulled out by means of the internal gear-and-rack drive, and the PFS soft protective cap at the front end of the injector pen is removed. Compared with existing products, the actions of removing the pen cap and removing the soft protective cap are dispensed with, thereby simplifying the operating procedure and greatly enhancing convenience for emergency treatment and saving rescue-critical time.

The present invention relates to a fixed-dose auto-injector pen. Other advantages, objects and features of the present invention will be embodied in part in the following description, and will in part be understood by those skilled in the art by study and practice of the present invention.

### BRIEF DESCRIPTION OF THE DRAWINGS

The accompanying drawings are provided for a further understanding of the present invention and constitute a part of this specification. Together with the embodiments of the present invention, they serve to explain the present invention and do not constitute a limitation thereof. In the drawings:
Fig. 1 is a schematic structural view of the present invention.
Fig. 2 is a schematic structural view of the pen body of the present invention.
Fig. 3 is an exploded view of the present invention.
Fig. 4 is a schematic view of the pen body of the present invention.
Fig. 5 is a schematic structural view of the present invention with the protective bolt pulled out.
Fig. 6 is a schematic structural view of the PFS sleeve container of the present invention.
Fig. 7 is a schematic structural view of the piston push rod of the present invention.
Fig. 8 is a schematic structural view of the clamp of the present invention.
Fig. 9 is a schematic structural view of the power assembly body of the present invention.
Fig. 10 is a schematic structural view of the elastic piece of the present invention.
Fig. 11 is a schematic structural view of the protective bolt of the present invention.
Fig. 12 is a schematic structural view of the protective shell of the present invention.
Fig. 13 is a schematic structural view of the rear cover of the present invention.
Fig. 14 is a schematic view of the pen barrel of the present invention.
Fig. 15 is a schematic view of the pen body of the present invention.
Fig. 16 is a schematic view of the present invention with the protective bolt pulled out.
Fig. 17 is a schematic structural view of the present invention during needle extension.
Fig. 18 is a schematic structural view of the PFS sleeve of the present invention.
Fig. 19 is a schematic view and a sectional view of the PFS of the present invention.
Fig. 20 is a schematic structural view of the moulding device of the present invention.
Fig. 21 is a schematic structural view of the detection mechanism of the present invention.
Fig. 22 is a schematic partial structural view of the buffer device of the present invention.
Fig. 23 is a schematic structural view of the buffer device of the present invention.
Fig. 24 is a schematic side structural view of the buffer device of the present invention.

Description of reference numerals: 1, pen barrel; 2, barrel cap; 3, pen body; 4, hook; 5, pen cap; 6, gear; 7, rack; 8, cap hook; 9, PFS sleeve container; 10, PFS sleeve; 11, PFS; 12, rear cover; 13, protective bolt; 14, power assembly body; 15, opening; 16, rear auxiliary spring; 17, insertion post; 18, arc-shaped piece; 19, sliding hole; 20, recess; 21, clamp; 23, connecting portion; 24, sliding portion; 25, protrusion slot; 26, push rod hole; 27, first slider slot; 28, second slider slot; 29, piston push rod; 30, push rod; 31, piston rod; 32, sliding block; 33, push rod groove; 34, main spring; 35, container body; 36, observation hole; 37, arc-shaped elastic piece; 38, second stop block; 39, container hook; 40, cylindrical barrel; 41, first stop block; 42, elastic-piece retaining slot; 43, elastic piece; 44, elastic-piece positioning groove; 45, support-platform groove; 46, support platform; 47, support arm; 48, negative-angle catch platform; 49, protective shell; 50, shell body; 51, catch-platform hole; 52, first rectangular hole; 53, second rectangular hole; 54, third rectangular hole; 55, arc-shaped trigger plate; 56, front auxiliary spring; 57, bolt body; 58, catch tab; 59, protective piece; 60, warning sticker; 61, drug viewing window; 62, soft protective cap; 63, detection hole; 64, pen body label; 101, protrusion; 102, step; 103, semicircular hole, narrower at the top and wider at the bottom; 104, inclined surface; 105, limiting step; 106, limiting piece; 107, inner step; 108, rectangular limiting frame.

### DETAILED DESCRIPTION OF EMBODIMENTS

The present invention is further described in detail below with reference to the accompanying drawings and the embodiments, so that those skilled in the art may carry it out by reference to the text of this specification.

It should be understood that terms such as "have", "contain" and "comprise" as used herein do not exclude the presence or addition of one or more other elements or combinations thereof.

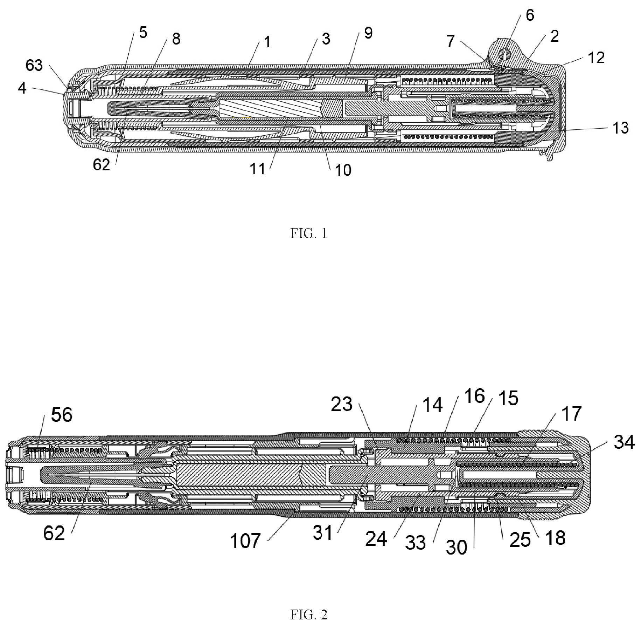

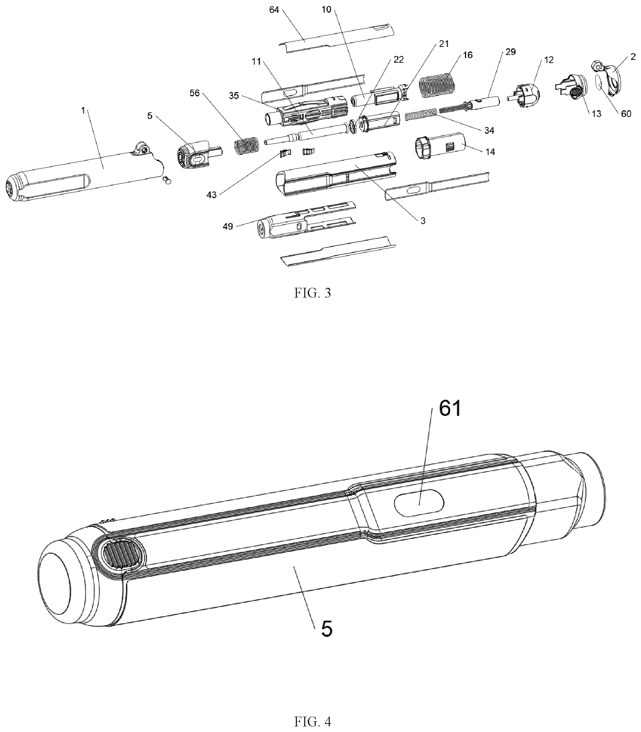

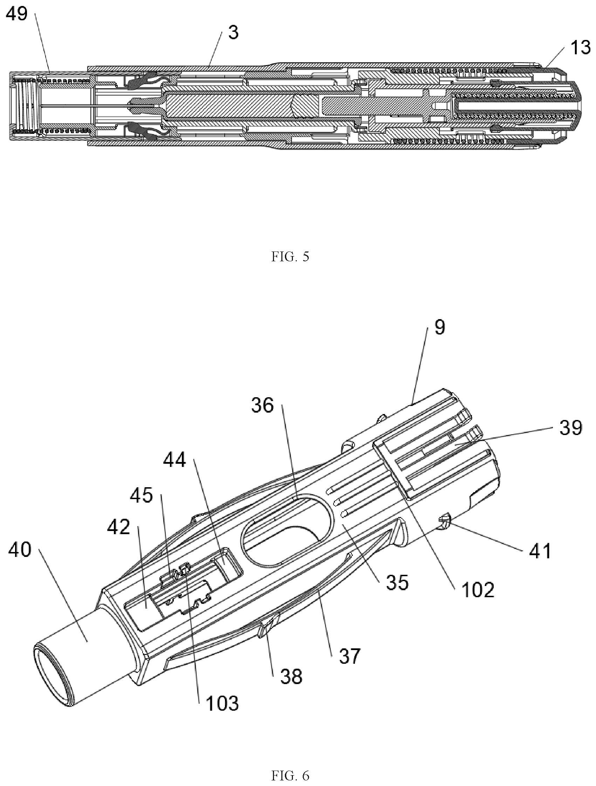

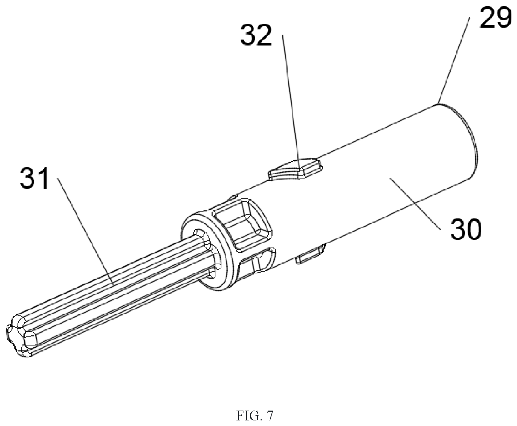

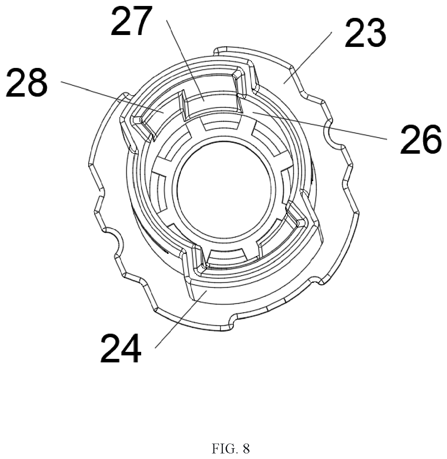

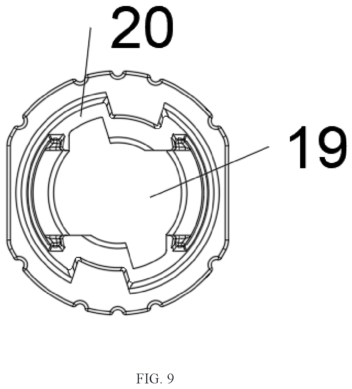

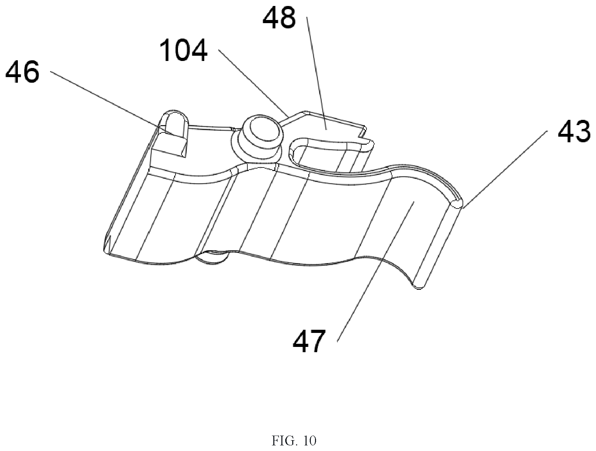

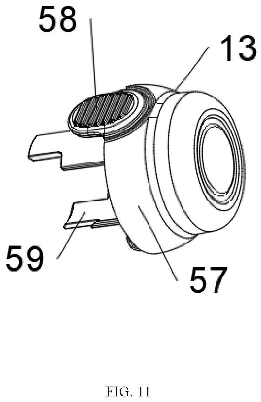

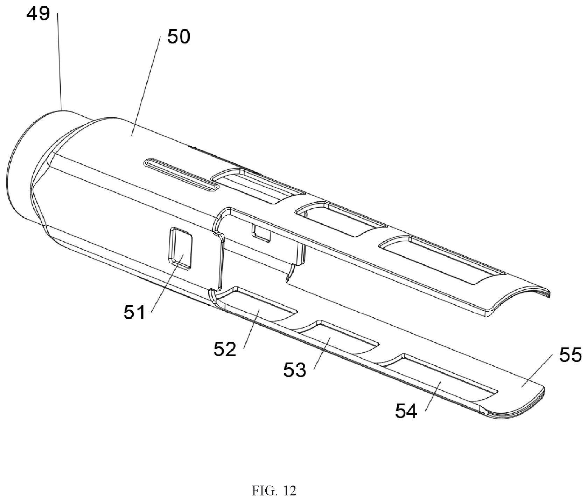

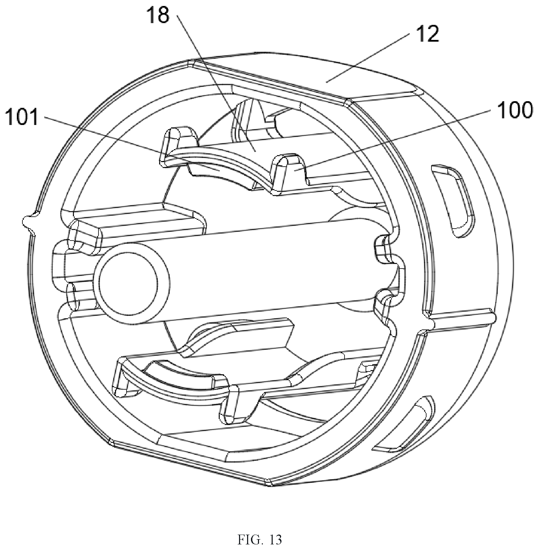

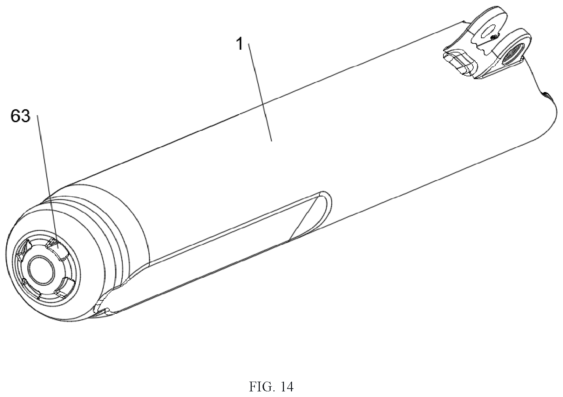

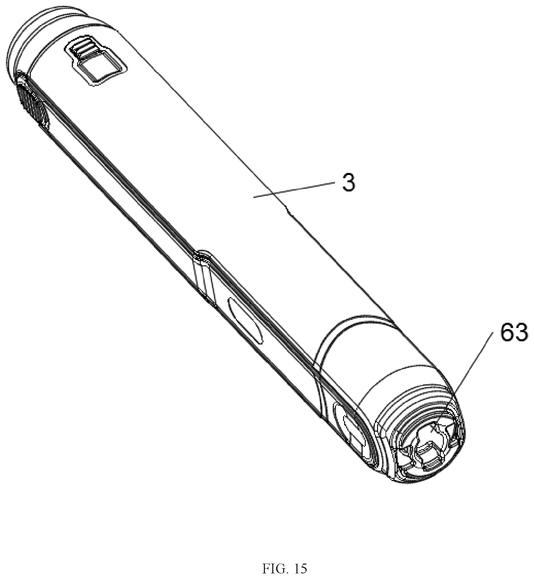

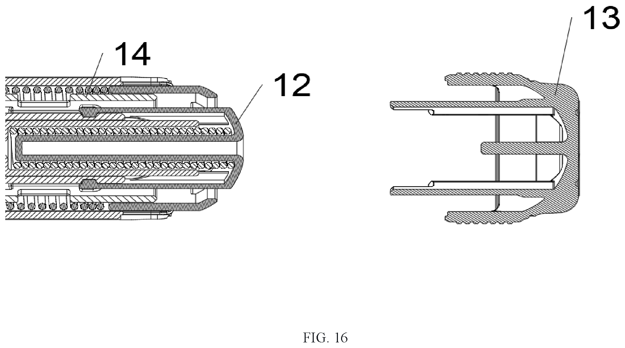

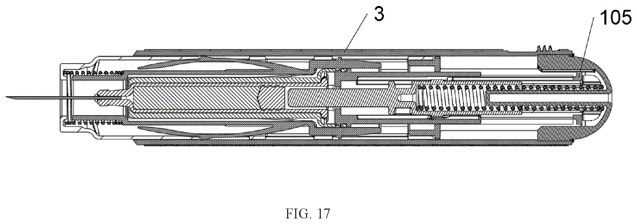

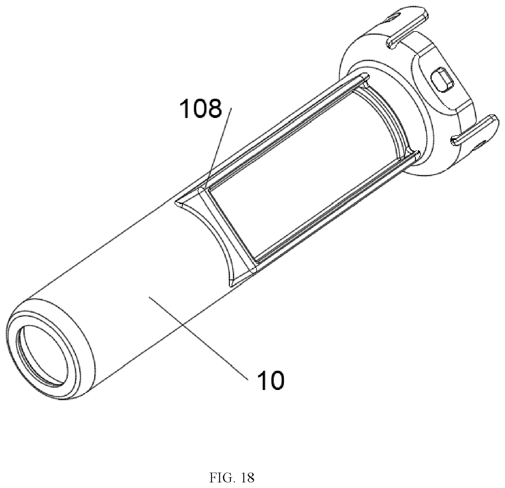

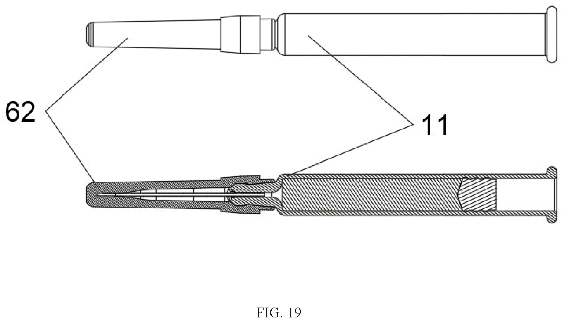

As shown in Figs. 1 to 19, the present invention provides a fixed-dose auto-injector pen, including:
a pen barrel 1, a pen body 3 being provided inside the pen barrel 1, one end of the pen body 3 being provided with a pen cap 5, one end of the pen barrel 1 being provided with a barrel cap 2, the other end of the pen body 3 being provided with a rear cover 12, and the rear cover 12 being provided with a protective bolt 13;
a PFS sleeve container 9 being provided in the pen body 3, the PFS sleeve container 9 being snap-fitted to the pen body 3, a PFS sleeve 10 being slidably provided in the PFS sleeve container 9, a PFS 11 being provided in the PFS sleeve 10, the PFS 11 being provided with a soft protective cap 62, a secondary power assembly being provided between the PFS sleeve 10 and the rear cover 12, and a trigger protection mechanism being provided on the pen body 3, the trigger protection mechanism being located between the pen cap 5 and the PFS sleeve container 9.

Working principle and advantageous effects of the above technical solution: In actual use, the barrel cap 2 is opened and the pen body 3 is withdrawn; during withdrawal, the pen barrel 1 takes off the pen cap 5, and the pen cap 5 pulls off the soft protective cap 62 at the same time. The protective bolt 13 is then pulled out horizontally, and the protective shell is pressed to actuate the trigger protection mechanism, whereupon the trigger protection mechanism triggers the secondary power assembly. The movement of the secondary power assembly drives the PFS sleeve 10 to slide inside the PFS sleeve container 9, so that the PFS 11 is pushed out and the injection is carried out. After the injection has been completed, the trigger protection mechanism rebounds and locks, thereby shielding the protruding needle and preventing the needle from remaining exposed to the outside, which would readily cause secondary injury to a user.

In one embodiment, the barrel cap 2 is provided with a gear 6, the pen body 3 is provided with a rack 7, and the gear 6 meshes with the rack 7;
the end of the pen barrel 1 remote from the barrel cap 2 is provided with a hook 4, the hook 4 being snap-fitted to a retaining slot of the pen cap 5; a plurality of cap hooks 8 are evenly provided inside the pen cap 5, and the cap hooks 8 engage with the soft protective cap 62.

Working principle and advantageous effects of the above technical solution: In actual use, during the opening of the barrel cap 2, as the barrel cap 2 rotates, the gear 6 and the rack 7 drive the pen body 3 to move a certain distance out of the pen barrel 1, thereby facilitating removal of the pen body 3. At the same time, the hook 4 holds the pen cap 5 in place, so that when the pen body 3 is pulled out, the cap hooks 8 can automatically pull off the soft protective cap 62. The soft protective cap is thus removed without any additional action being required before use, which simplifies the operating procedure, avoids operating errors and improves efficiency of use.

In one embodiment, an inner wall of the rear cover 12 is provided with an insertion post 17, and two arc-shaped pieces 18 are symmetrically provided on the inner wall of the rear cover 12, the two adjacent arc-shaped pieces 18 being located on either side of the insertion post 17; the side of each arc-shaped piece 18 close to the insertion post 17 is provided with a protrusion 101, and the side of each arc-shaped piece 18 remote from the insertion post 17 is provided with two limiting pieces 106.

A plurality of limiting steps 105 are provided on the inner wall of the rear cover 12.

Working principle and advantageous effects of the above technical solution: The rear cover 12 is fixedly connected to the pen body 3 and provides a power support point for the secondary power assembly. At the same time, protrusions 101 provided on the arc-shaped pieces 18 can exert a restraining action on the secondary power assembly, preventing the secondary power assembly from actuating without being triggered; they can also cooperate with the protective bolt 13 to lock the secondary power assembly. As long as the protective bolt 13 has not been pulled out, the trigger mechanism cannot be triggered even if the protective shell 49 is pressed, which improves the stability of the injector pen as a whole.

In one embodiment, the secondary power assembly includes:
a power assembly body 14, the power assembly body 14 being slidably connected to an inner wall of the pen body 3, the end of the power assembly body 14 remote from the rear cover being provided with a recess 20, the bottom end of an inner wall of the recess 20 being provided with a sliding hole 19, the power assembly body 14 being provided with two openings 15, a rear auxiliary spring 16 being provided between the power assembly body 14 and the rear cover 12, a clamp 21 being provided in the power assembly body 14, and a piston push rod 29 being provided in the clamp 21;
the clamp 21 includes:
   a connecting portion 23, the connecting portion 23 being slidably connected in the recess 20, the connecting portion 23 being fixedly snap-fitted to the PFS sleeve 10, the connecting portion 23 being provided with a sliding portion 24, the sliding portion 24 extending into the sliding hole 19, an outer wall of the sliding portion 24 being provided with two protrusion slots 25, the protrusions 101 being engaged in the protrusion slots 25, a push rod hole 26 being provided in the sliding portion 24, an inner wall of the push rod hole 26 being provided with two first slider slots 27, an inner side wall of each first slider slot 27 being provided with a second slider slot 28, and an inner-wall inclined surface of the second slider slot 28 being a guiding inclined surface;
   the piston push rod 29 includes:
      a push rod 30, the push rod 30 being slidably connected in the push rod hole 26, an outer wall of the push rod 30 being provided with two sliding blocks 32, the sliding blocks 32 being located in the second slider slots 28, a push rod groove 33 being provided in the push rod 30, the insertion post 17 extending into the push rod groove 33, a main spring 34 being provided between the push rod groove 33 and the rear cover 12, and the main spring 34 being fitted over the insertion post 17;
      a piston rod 31, one end of the piston rod 31 being connected to the push rod 30, and the other end of the piston rod 31 passing through the connecting portion 23 and extending into the PFS 11.

Working principle and advantageous effects of the above technical solution: In actual use, after the protective bolt 13 has been pulled out, the trigger protection mechanism is pressed, and the trigger protection mechanism pushes the power assembly body 14 to move in the direction in which the rear auxiliary spring 16 is compressed, so that the power assembly body 14 and the clamp 21 slide relative to each other. When the openings 15 slide to a position above the protrusions 101 of the arc-shaped pieces 18, the arc-shaped pieces 18 are released from the restraint of the power assembly body 14. Under the action of the main spring 34, the main spring 34 drives the clamp 21, by means of the sliding blocks 32 on the push rod 30, to move in the direction in which the main spring 34 extends; at the same time, the clamp 21 drives the PFS sleeve 10 to move, so that the needle of the PFS 11 is pushed out and pierces the injection site on the human body. After the clamp 21 has driven the PFS sleeve 10 a certain distance, the connecting portion 23 of the clamp 21 abuts against the PFS sleeve container 9, so that the clamp 21 can no longer drive the needle of the PFS 11 further into the human body; at this moment the needle tip is at the optimum injection position. When the clamp 21 can no longer move, then, because the inner-wall inclined surface of the second slider slots 28 is a guiding inclined surface, the sliding blocks 32 slide along the guiding inclined surface and slide into the first slider slots 27, so that the main spring 34 causes the push rod 30 and the clamp 21 to slide relative to each other, whereby the piston rod 31 moves in the direction in which the main spring 34 extends and thereby pushes the piston inside the PFS 11 to move, expelling the liquid medicament in the PFS 11 and thus completing the injection. Because the injection is completed under the drive of the secondary power assembly, the injection is designed in such a way that the needle is advanced first and the liquid medicament is only then expelled; this can ensure the accuracy of the dose of liquid medicament administered and the therapeutic efficacy of intramuscular injection treatment, and at the same time ensures that at every injection the drug is injected at the optimum injection depth.

In one embodiment, the PFS sleeve container 9 includes:
a container body 35, the PFS sleeve 10 being located in the container body 35, one end of the container body 35 being provided with a cylindrical barrel 40, the other end of the container body 35 being provided with a container hook 39, the container hook 39 being snap-fitted to the pen body 3, the container body 35 being provided with two first stop blocks 41, two arc-shaped elastic pieces 37 being symmetrically provided on the container body 35, and the arc-shaped elastic pieces 37 being provided with second stop blocks 38;
two steps 102 being provided, above and below, where the tail end of the container body 35 joins the tail end of the container hook 39, and the steps 102 engaging with the inner steps 107 inside the pen body 3;
elastic-piece retaining slots 42, two elastic-piece retaining slots 42 being symmetrically provided on the container body 35, the elastic-piece retaining slots 42 being located between the cylindrical barrel 40 and the container hook 39, an elastic-piece positioning groove 44 being provided in each elastic-piece retaining slot 42, a support-platform groove 45 being provided in each elastic-piece retaining slot 42, and an elastic piece 43 being provided in each elastic-piece retaining slot 42;
two semicircular holes 103, each narrower at the top and wider at the bottom, being provided in each elastic-piece retaining slot 42;
the elastic piece 43 includes:
   a support platform 46, the support platform 46 being rotatably connected within the semicircular hole 103, which is narrower at the top and wider at the bottom, inside the elastic-piece retaining slot 42 by means of a rotating shaft, one end of the support platform 46 extending into the support-platform groove 45, and the other end of the support platform 46 being provided with a support arm 47 and a negative-angle catch platform 48, the negative-angle catch platform 48 being provided with an inclined surface 104.

Working principle and advantageous effects of the above technical solution: In actual use, a retaining slot for limiting the connecting portion 23 of the clamp 21 is provided inside the container body 35, so that once the connecting portion 23 has entered the container body 35 and travelled a certain distance, it can no longer move further forward.

By means of the container hook 39 provided, the container body 35 can be quickly snapped into the interior of the pen body, which facilitates rapid assembly during production. At the same time, the elastic piece 43 provided can cooperate with the trigger protection mechanism.

Because the elastic piece 43 has support platforms 46 on both sides, the rotating shafts on both sides of the elastic piece can only rotate clockwise inside the shaft holes of the container body. When the protective shell is pressed for actuation, the catch-platform hole 51 in the protective shell presses against the inclined surface 104 of the negative-angle catch platform of the elastic piece 43, forcing the elastic piece to rotate clockwise about the shaft, so that the negative-angle catch platform 48 of the elastic piece disengages from its hooked engagement with the catch-platform hole 51. The PFS sleeve 10 then moves to a position below the elastic piece 43, and the outer cylindrical surface of the PFS sleeve 10 presses against the underside of the elastic piece 43, so that the negative-angle catch platform 48 of the elastic piece 43 can no longer hook onto the protective shell, and the protective shell can spring out to provide protection.

In one embodiment, the trigger protection mechanism includes:
a protective shell 49 slidably connected inside the pen body 3, the protective shell 49 being located between the secondary power assembly and the pen cap 5, the protective shell 49 including:
a shell body 50, one end of the shell body 50 being provided with a through hole, the shell body 50 being provided with a plurality of catch-platform holes 51, the catch-platform holes 51 being adapted to the negative-angle catch platforms 48, the other end of the shell body 50 being symmetrically provided with two arc-shaped trigger plates 55, each arc-shaped trigger plate 55 being provided with a first rectangular hole 52, a second rectangular hole 53 and a third rectangular hole 54 arranged side by side, the arc-shaped trigger plates 55 abutting against the power assembly, the first stop blocks 41 being located in the third rectangular holes 54, and the second stop blocks 38 being located in the first rectangular holes 52;
the container body 35 being located between the two adjacent arc-shaped trigger plates 55;
a front auxiliary spring 56, the front auxiliary spring 56 being provided inside the shell body 50, the front auxiliary spring 56 being located between the inner wall of the shell body 50 and the container body 35, and the front auxiliary spring 56 being arranged close to the through hole.

Working principle and advantageous effects of the above technical solution: In actual use, during the injection, the front end of the protective shell 49 contacts the injection site and is compressed, which causes the protective shell 49 and the pen body 3 to slide relative to each other. During this process, because of the negative-angle design of the negative-angle catch platform 48, the negative-angle catch platform 48 withdraws from the catch-platform hole 51 when it is compressed, and the support arm 47 of the elastic piece 43 is compressed and deforms, so that the protective shell 49 moves in the direction in which the rear auxiliary spring 16 is compressed. The trigger plates 55 of the protective shell 49 push the power assembly body 14 to move, thereby triggering the secondary power assembly, and the injection begins. During the movement of the trigger plates 55, the second stop blocks 38 slide in the first rectangular holes 52 and the first stop blocks 41 slide in the third rectangular holes 54; after sliding a certain distance, the first stop blocks 41 and the second stop blocks 38 abut against the edges of the third rectangular holes 54 and of the first rectangular holes 52 respectively, so that the protective shell 49 cannot continue to move inward, which prevents the injection position from being too deep. After the injection has been completed, when the needle of the PFS 11 is withdrawn, the front auxiliary spring 56 undergoes restoring deformation and drives the protective shell 49 to move in the direction away from the PFS sleeve container 9. During this process, the second stop blocks 38 press the arc-shaped elastic pieces 37 and then enter the second rectangular holes 53, and the first stop blocks 41 withdraw from the third rectangular holes 54, thereby propping up the protective shell 49 and preventing the protective shell 49 from moving in the direction of the PFS sleeve container 9 after the injector pen is removed from the injection site upon completion of injection. The withdrawn needle is thus confined inside the protective shell 49, preventing the needle from being exposed and causing injury to the user.

When the protective shell is pressed for actuation of the first-stage needle advance, the protective shell is pressed and the tail end of the protective shell pushes the power assembly body to move; the power assembly body stops when it reaches the position of the limiting step 105, and the limiting step 105 limits the pressing distance.

In one embodiment, the protective bolt 13 includes:
a bolt body 57, the bolt body 57 being of a cap-shaped structure, an outer wall of the bolt body 57 being provided with two catch tabs 58, the catch tabs 58 being racetrack-shaped, the surfaces of the catch tabs 58 being provided with anti-slip patterns, and the bolt body 57 being connected to the pen body 3 by means of the catch tabs; and
protective pieces 59, two protective pieces 59 being symmetrically provided on an inner wall of the bolt body 57, the protective pieces 59 being located between the power assembly body 14 and the arc-shaped pieces 18, and the protective pieces 59 being further located between two adjacent limiting pieces 106.

Working principle and advantageous effects of the above technical solution: The catch tabs 58 are provided with anti-slip patterns, which facilitates removal of the protective bolt during use. At the same time, when the protective bolt 13 has not been pulled out, the protective pieces 59 of the protective bolt 13 are located between the arc-shaped pieces 18 and the power assembly body 14, further restraining the protrusions 101 on the arc-shaped pieces 18, so that the secondary power assembly cannot be triggered; moreover, the limiting pieces 106 on the arc-shaped pieces 18 provide positional limitation for the protective pieces 59. The upper side of the arc-shaped pieces is subject to the limiting action of the inner wall of the power assembly body 14, so that even if the protective bolt has been removed, the arc-shaped pieces 18 are still restrained by the inner wall of the sleeve of the power assembly body 14, and actuation can only be achieved by pressing the protective shell.

In one embodiment, the protective bolt 13 is provided with a warning sticker 60, and the pen body 3 is provided with a plurality of pen body labels 64.

Working principle and advantageous effects of the above technical solution: By virtue of the design of the above structure, the warning sticker 60 and the pen body labels 64 provided make it convenient for the user to be informed in good time of the various information about the injector pen.

In one embodiment, a drug viewing window 61 is provided on the pen body 3, the container body 35 is provided with an observation hole 36, and the position of the observation hole 36 corresponds to that of the drug viewing window 61.

Working principle and advantageous effects of the above technical solution: The drug viewing window 61 and the observation hole 36 provided make it convenient for the user to observe the drug, to confirm whether the drug has become turbid, discoloured or deteriorated, and to determine whether the injector pen needs to be replaced.

In one embodiment, the end of the pen barrel 1 remote from the barrel cap 2 and the pen cap 5 are each provided with a detection hole 63, and the positions of the detection holes on the pen barrel 1 and on the pen cap 5 correspond to each other.

Working principle and advantageous effects of the above technical solution: The detection holes 63 provided allow a detector to be inserted, while the pen body 3 is inside the pen barrel 1, in order to push the trigger protection mechanism and thereby determine whether the power assembly is locked; to determine whether the accumulated spring force of the front auxiliary spring and the rear auxiliary spring is within specification; and to determine whether any of the mating parts slide unsmoothly and thus give rise to the problem of excessive resistance.

In one embodiment, the PFS sleeve 10 is cylindrical, and two rectangular limiting frames 108 are provided on the outer wall of the PFS sleeve 10, the rectangular limiting frames 108 being slidably connected inside the PFS sleeve container 9.

Working principle and advantageous effects of the above technical solution: The rectangular limiting frames 108 provide a positional limitation for the PFS sleeve 10.

In one embodiment, the pre-filled syringe (PFS) is a conventional disposable, standard pre-filled syringe; only the needle needs to be replaced, which greatly reduces the demands of the production process, raises the yield and lowers costs. In addition, because of its small volume, the pre-filled syringe takes up no space, is able to deliver an accurate drug dose reliably, and carries a low risk of contamination.

### Advantages:

A conventional slender-type PFS is used and only the needle is replaced, which reduces to a very great extent the processing difficulty of the drug packaging material and shortens the procurement cycle.

The conventional slender-type PFS is sealed with a silicone soft protective cap before use, which ensures the sealing integrity of the liquid medicament and that it is not contaminated; the soft protective cap is removed without any additional action being required before use.

The two-step operating method, identical to that of the originator pen, suits the habitual operation of patients and medical personnel and reduces the occurrence of usage errors.

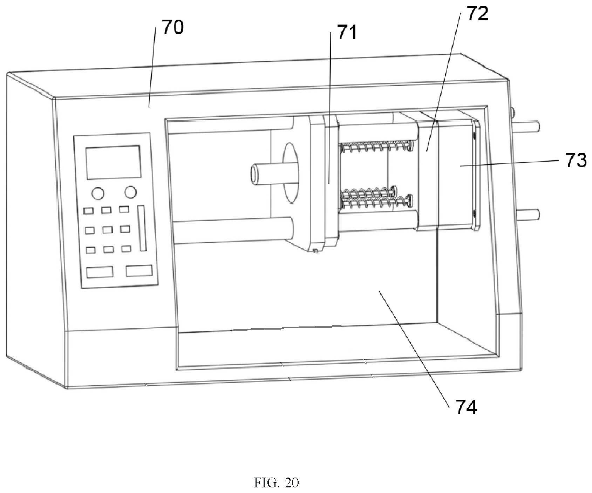

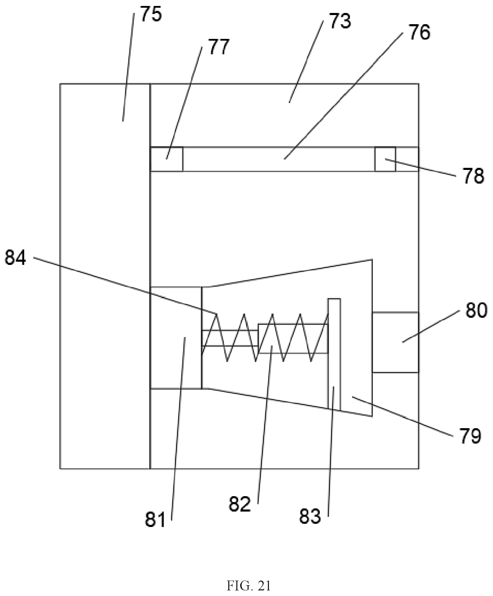

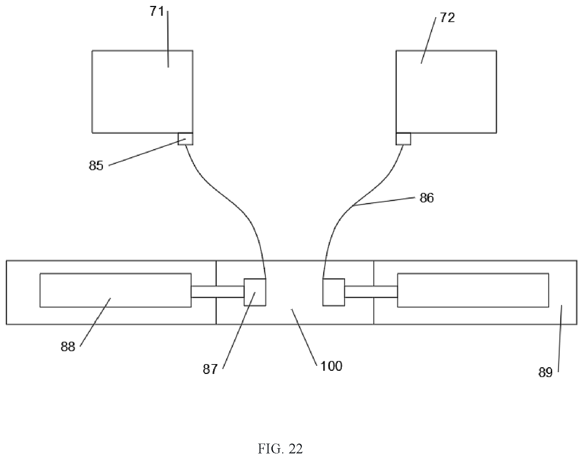

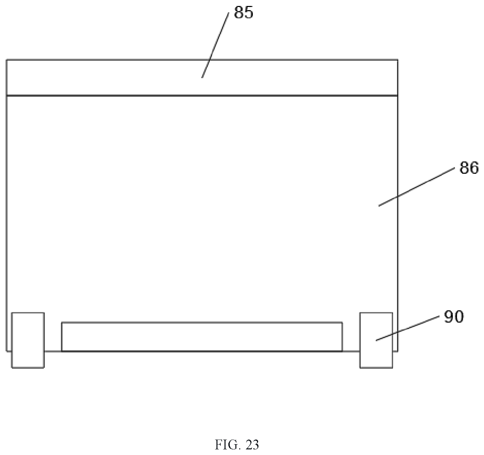

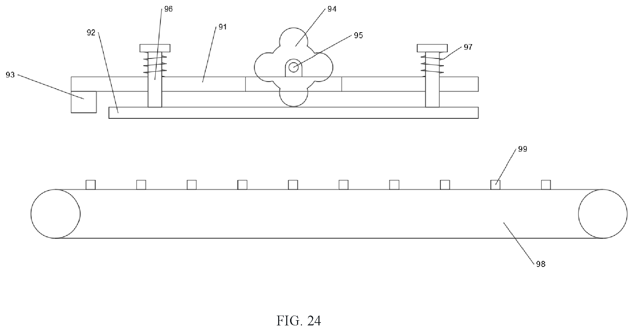

As shown in Figs. 20 to 24, the present invention further provides a production process for a fixed-dose auto-injector pen, including the following steps:
Step 1: replacing a mould on a moulding device in accordance with the required component;
Step 2: starting the moulding device, injection-moulding the corresponding component, and cleaning the surface of the component;
Step 3: selecting the corresponding components and assembling them, mounting a trigger protection mechanism, a secondary power assembly and a PFS onto a PFS sleeve container, mounting these together with a rear cover and a protective bolt into a pen body, fitting a pen cap on, engaging a soft protective cap of the PFS with cap hooks, and then applying pen body labels; and
Step 4: inserting the assembled pen body into a pen barrel so that the pen barrel is connected to the pen cap, and fitting a barrel cap, whereby production is completed.

In one embodiment, the moulding device includes:
an injection moulding machine 70, an injection moulding chamber 74 being provided in the injection moulding machine 70, a movable mould 71 and a fixed mould 72 being provided in the injection moulding chamber 74, the fixed mould 72 being connected to an inner wall of the injection moulding chamber 74 by means of a mounting block 73, a moulding cavity 75 being provided in each of the fixed mould 72 and the movable mould 71, and the injection moulding machine 70 being electrically connected to a controller;
a detection mechanism, the detection mechanism being provided in the mounting block 73, the detection mechanism including:
   an air intake pipe 76, one end of the air intake pipe 76 being connected to the moulding cavity 75 and the other end of the air intake pipe 76 passing through the mounting block 73, a pressure valve 77 and an air flow sensor 78 being provided in the air intake pipe 76, and the air flow sensor 78 being electrically connected to the controller;
   a conical chamber 79, the conical chamber 79 being provided in the mounting block 73, the conical chamber 79 being located below the air intake pipe 76, a blocking plug 81 being slidably connected in the conical chamber 79, one side of the blocking plug 81 being flush with the inner wall of the moulding cavity 74, a fixing plate 83 being provided on an inner wall of the conical chamber 79, and a detection spring 84 and a telescopic rod 82 being provided between the fixing plate 83 and the blocking plug 81; and
   an air extraction pipe 80, one end of the air extraction pipe 80 being connected to the conical chamber 79 and the other end of the air extraction pipe 80 being connected to an air extractor.

Working principle and advantageous effects of the above technical solution: After the fixed mould 72 and the movable mould 71 have been closed together, the air extractor is started; the air extractor causes air to be discharged from the conical chamber 79 through the air extraction pipe 80, so that a negative pressure is formed in the conical chamber 79 and the blocking plug 81 moves in the direction in which the spring 84 is compressed, whereby the air in the moulding cavity 75 is drawn away and a negative pressure is formed in the moulding cavity 75. When the moulding cavity 75 is in a well-sealed state, air passes through the air intake pipe 76 into the moulding cavity 75, and the air flow sensor 78 then detects a value, which indicates that the air-tightness of the moulding cavity 75 is good; the injection moulding machine then injects material into the moulding cavity 75 and the component is produced. When no value is detected, this indicates that the sealing of the moulding cavity 75 is poor; the injection moulding machine will not carry out injection moulding, and at the same time issues a reminder prompting the operator to carry out an inspection. This avoids the situation in which imprecise closing of the movable mould and the fixed mould, or deformation of the mould, causes the liquid plastic injected into the moulding cavity 75 to leak outward and thus results in failure of the component moulding, thereby saving costs.

In one embodiment, a buffer device is further provided in the injection moulding chamber 74, the buffer device including:
a belt conveyor 98, the belt conveyor 98 being provided in the injection moulding chamber 74, and cross bars 99 being evenly provided on the belt conveyor 98;
a fixing plate 91, two ends of the fixing plate 91 being arranged in parallel in the injection moulding chamber 74 by means of struts, an adjusting plate 92 being arranged in parallel at the bottom end of the fixing plate 91, and a photographic vision detector being provided at the bottom end of the fixing plate 91;
a plurality of T-shaped rods 96, one end of each of the T-shaped rods 96 being connected to the top end of the adjusting plate 92, the other end of each T-shaped rod 96 passing through the fixing plate 91, and an adjusting spring 97 being provided between each T-shaped rod 96 and the fixing plate 91;
a rotating rod 95, one end of the rotating rod 95 being connected to a motor, the motor being provided at the top end of the fixing plate 91, the other end of the rotating rod 95 being connected to a pressing wheel 94, the rotating rod 95 rotatably passing through an auxiliary block, the auxiliary block being fixedly provided at the top end of the fixing plate 91, and the pressing wheel 94 passing through the fixing plate 91 and abutting against the top end of the adjusting plate 92;
a mounting block 89, the mounting block 89 being fixedly provided on the inner wall of the injection moulding chamber 74, a component through-hole 100 being provided in the mounting block 89, two electric telescopic rods 88 being symmetrically provided on the inner wall of the component through-hole 100, and a lower connecting strip 87 being provided on each electric telescopic rod 88;
upper connecting strips 85, an upper connecting strip 85 being provided at the bottom end of each of the fixed mould 72 and the movable mould 71;
a flexible cloth bag 86, two sides of the top end of the flexible cloth bag 86 being connected respectively to two adjacent upper connecting strips 85, and two sides of the bottom end of the flexible cloth bag 86 being connected respectively to two adjacent lower connecting strips 87; and
a plurality of air jet pipes 90, one end of each of the air jet pipes 90 being connected to the inner wall of the bottom end of the flexible cloth bag 86, and the other end of each air jet pipe 90 being connected to a fan.

Working principle and advantageous effects of the above technical solution: In actual use, when the fixed mould 72 and the movable mould 71 separate after processing has been completed, the movable mould 71 drives one side of the top end of the flexible cloth bag 86 to move, so that the flexible cloth bag 86 opens. Once the mould has opened, the component inside falls and enters the flexible cloth bag 86. During its fall, the component is first decelerated and cleaned by the gas jetted from the air jet pipes 90, and at the same time the gas further lowers the temperature of the component, so that the interior of the component and its detailed portions are rapidly cooled and set, whereby the component is completely set and deformation is avoided. When the set component comes into contact with the flexible cloth bag 86, the flexible cloth bag 86 deforms slightly, thereby buffering the falling component and preventing the component from falling directly onto the conveying mechanism and being deformed, which improves the good-product yield of the moulding device. After the components have fallen, the gas jetted from the air jet pipes 90 washes the inner walls of the mould through the flexible cloth bag 86, so that residues inside the mould do not affect the production of subsequent components during later processing; moreover, the flexible cloth bag 86 is arranged closely against the mould, preventing components from scattering. Once the components have finished falling, the electric telescopic rods 88 retract, so that the bottom end of the flexible cloth bag 86 opens and the components fall onto the belt conveyor 98 and are conveyed, then undergoing visual inspection as they pass the vision detector. During this process, the motor drives the pressing wheel 94 to rotate by means of the rotating rod 95, and as the pressing wheel 94 rotates it drives the adjusting plate 92 to move up and down. This cycle is repeated, so that each part undergoes fine adjustment of its centre-of-gravity position in sequence, whereby the positions of the components in the photographs output by the vision detector 93 approximate those in the standard file. This facilitates comparison and thus facilitates the screening out of defective products.

In the description of the present invention, it should be understood that the orientations or positional relationships indicated by the terms "centre", "longitudinal", "transverse", "length", "width", "thickness", "upper", "lower", "front", "rear", "left", "right", "vertical", "horizontal", "top", "bottom", "inner", "outer", "clockwise", "anticlockwise", "axial", "radial", "circumferential" and the like are based on the orientations or positional relationships shown in the accompanying drawings and are merely intended to facilitate the description of the present invention and to simplify that description, rather than indicating or implying that the device or element referred to must have a particular orientation or be constructed and operated in a particular orientation; they may therefore not be construed as limiting the present invention.

In the present invention, unless otherwise expressly specified and limited, the terms "mount", "join", "connect", "fix" and the like are to be understood in a broad sense. For example, a connection may be a fixed connection, a detachable connection, or an integral formation; it may be a mechanical connection, an electrical connection, or a connection permitting mutual communication; it may be a direct connection, or an indirect connection by means of an intermediate medium; and it may be an internal communication between two elements or an interaction relationship between two elements, unless otherwise expressly limited. For a person of ordinary skill in the art, the specific meanings of the above terms in the present invention may be understood according to the specific circumstances.

Although the embodiments of the present invention have been disclosed as above, they are not limited merely to the applications set forth in the description and the embodiments; they may be applied entirely to various fields suitable for the present invention. For those familiar with the art, further modifications may readily be made. Therefore, without departing from the general concept defined by the claims and the scope of their equivalents, the present invention is not limited to the specific details and to the illustrations shown and described herein.

## Claims

1. A fixed-dose auto-injector pen, comprising:
a pen barrel (1), a pen body (3) being provided inside the pen barrel (1), one end of the pen body (3) being provided with a pen cap (5), one end of the pen barrel (1) being provided with a barrel cap (2), the other end of the pen body (3) being provided with a rear cover (12), and the rear cover (12) being provided with a protective bolt (13);
a pre-filled syringe (PFS) sleeve container (9) being provided in the pen body (3), the PFS sleeve container (9) being snap-fitted to the pen body (3), a PFS sleeve (10) being slidably provided in the PFS sleeve container (9), a PFS (11) being provided in the PFS sleeve (10), the PFS (11) being provided with a soft protective cap (62), a secondary power assembly being provided between the PFS sleeve (10) and the rear cover (12), and a trigger protection mechanism being provided on the pen body (3), the trigger protection mechanism being located between the pen cap (5) and the PFS sleeve container (9).

2. The fixed-dose auto-injector pen according to claim 1, wherein
the barrel cap (2) is provided with a gear (6), the pen body (3) is provided with a rack (7), and the gear (6) meshes with the rack (7);
the end of the pen barrel (1) remote from the barrel cap (2) is provided with a hook (4), the hook (4) being snap-fitted to a retaining slot of the pen cap (5); a plurality of cap hooks (8) are evenly provided inside the pen cap (5), and the cap hooks (8) engage with the soft protective cap (62).

3. The fixed-dose auto-injector pen according to claim 1, wherein
an inner wall of the rear cover (12) is provided with an insertion post (17), and two arc-shaped pieces (18) are symmetrically provided on the inner wall of the rear cover (12), the two adjacent arc-shaped pieces (18) being located on either side of the insertion post (17); the side of each arc-shaped piece (18) close to the insertion post (17) is provided with a protrusion (101), and the side of each arc-shaped piece (18) remote from the insertion post (17) is provided with two limiting pieces (106).

4. The fixed-dose auto-injector pen according to claim 3, wherein
the secondary power assembly comprises:
a power assembly body (14), the power assembly body (14) being slidably connected to an inner wall of the pen body (3), the end of the power assembly body (14) remote from the rear cover being provided with a recess (20), the bottom end of an inner wall of the recess (20) being provided with a sliding hole (19), the power assembly body (14) being provided with two openings (15), a rear auxiliary spring (16) being provided between the power assembly body (14) and the rear cover (12), a clamp (21) being provided in the power assembly body (14), and a piston push rod (29) being provided in the clamp (21);
the clamp (21) comprises:
a connecting portion (23), the connecting portion (23) being slidably connected in the recess (20), the connecting portion (23) being fixedly snap-fitted to the PFS sleeve (10), the connecting portion (23) being provided with a sliding portion (24), the sliding portion (24) extending into the sliding hole (19), an outer wall of the sliding portion (24) being provided with two protrusion slots (25), protrusions (101) being engaged in the protrusion slots (25), a push rod hole (26) being provided in the sliding portion (24), an inner wall of the push rod hole (26) being provided with two first slider slots (27), an inner side wall of each first slider slot (27) being provided with a second slider slot (28), and an inner-wall inclined surface of the second slider slot (28) being a guiding inclined surface;
the piston push rod (29) comprises:
a push rod (30), the push rod (30) being slidably connected in the push rod hole (26), an outer wall of the push rod (30) being provided with two sliding blocks (32), the sliding blocks (32) being located in the second slider slots (28), a push rod groove (33) being provided in the push rod (30), the insertion post (17) extending into the push rod groove (33), a main spring (34) being provided between the push rod groove (33) and the rear cover (12), and the main spring (34) being fitted over the insertion post (17); and
a piston rod (31), one end of the piston rod (31) being connected to the push rod (30), and the other end of the piston rod (31) passing through the connecting portion (23) and extending into the PFS (11).

5. The fixed-dose auto-injector pen according to claim 1, wherein
the PFS sleeve container (9) comprises:
a container body (35), the PFS sleeve (10) being located in the container body (35), one end of the container body (35) being provided with a cylindrical barrel (40), the other end of the container body (35) being provided with a container hook (39), the container hook (39) being snap-fitted to the pen body (3), the container body (35) being provided with two first stop blocks (41), two arc-shaped elastic pieces (37) being symmetrically provided on the container body (35), and the arc-shaped elastic pieces (37) being provided with second stop blocks (38);
two steps (102) being provided, above and below, where the tail end of the container body (35) joins the tail end of the container hook (39), and the steps (102) engaging with inner steps (107) inside the pen body (3);
elastic-piece retaining slots (42), two elastic-piece retaining slots (42) being symmetrically provided on the container body (35), the elastic-piece retaining slots (42) being located between the cylindrical barrel (40) and the container hook (39), an elastic-piece positioning groove (44) being provided in each elastic-piece retaining slot (42), a support-platform groove (45) being provided in each elastic-piece retaining slot (42), and an elastic piece (43) being provided in each elastic-piece retaining slot (42);
two semicircular holes (103), each narrower at the top and wider at the bottom, being provided in each elastic-piece retaining slot (42);
the elastic piece (43) comprises:
a support platform (46), the support platform (46) being rotatably connected within the semicircular hole (103) , which is narrower at the top and wider at the bottom, inside the elastic-piece retaining slot (42) by means of a rotating shaft, one end of the support platform (46) extending into the support-platform groove (45), and the other end of the support platform (46) being provided with a support arm (47) and a negative-angle catch platform (48).

6. The fixed-dose auto-injector pen according to claim 5, wherein
the trigger protection mechanism comprises:
a protective shell (49) slidably connected inside the pen body (3), the protective shell (49) being located between the secondary power assembly and the pen cap (5), the protective shell (49) comprising:
a shell body (50), one end of the shell body (50) being provided with a through hole, the shell body (50) being provided with a plurality of catch-platform holes (51), the catch-platform holes (51) being adapted to the negative-angle catch platforms (48), the other end of the shell body (50) being symmetrically provided with two arc-shaped trigger plates (55), each arc-shaped trigger plate (55) being provided with a first rectangular hole (52), a second rectangular hole (53) and a third rectangular hole (54) arranged side by side, the arc-shaped trigger plates (55) abutting against the power assembly, the first stop blocks (41) being located in the third rectangular holes (54), and the second stop blocks (38) being located in the first rectangular holes (52);
the container body (35) being located between the two adjacent arc-shaped trigger plates (55);
a front auxiliary spring (56), the front auxiliary spring (56) being provided inside the shell body (50), the front auxiliary spring (56) being located between the inner wall of the shell body (50) and the container body (35), and the front auxiliary spring (56) being arranged close to the through hole.

7. The fixed-dose auto-injector pen according to claim 6, wherein
the protective bolt (13) comprises:
a bolt body (57), the bolt body (57) being of a cap-shaped structure, an outer wall of the bolt body (57) being provided with two catch tabs (58), the catch tabs (58) being racetrack-shaped, the surfaces of the catch tabs (58) being provided with anti-slip patterns, and the bolt body (57) being connected to the pen body (3) by means of the catch tabs; and
protective pieces (59), two protective pieces (59) being symmetrically provided on an inner wall of the bolt body (57), the protective pieces (59) being located between the power assembly body (14) and the arc-shaped pieces (18), and the protective pieces (59) being further located between two adjacent limiting pieces (106).

8. The fixed-dose auto-injector pen according to claim 1, wherein
the protective bolt (13) is provided with a warning sticker (60), and the pen body (3) is provided with a plurality of pen body labels (64).

9. The fixed-dose auto-injector pen according to claim 1, wherein
a drug viewing window (61) is provided on the pen body (3), the container body (35) is provided with an observation hole (36), and the position of the observation hole (36) corresponds to that of the drug viewing window (61).

10. The fixed-dose auto-injector pen according to claim 1, wherein
the end of the pen barrel (1) remote from the barrel cap (2) and the pen cap (5) are each provided with a detection hole (63), and the positions of the detection holes (63) on the pen barrel (1) and on the pen cap (5) correspond to each other.
